# EUROPEAN PATENT APPLICATION

(11) **EP 0 639 585 A1**
(43) Date of publication of application: **22.02.1995**
(21) Application number: 94112266.5
(22) Date of filing: 05.08.1994
(51) Int. Cl.: C07K 5/06, C07K 5/02, C07K 5/08

(54) **Elastase inhibitor**

(30) Priority: 20.08.1993 JP 228173/93
(71) Applicant: BANYU PHARMACEUTICAL CO., LTD., Chuo-ku Tokyo 103 (JP); INSTITUTE OF MICROBIAL CHEMISTRY, Shinagawa, Tokyo (JP)
(72) Inventor: Takeuchi, Tomio, New Fuji mansion 701A, Shinagawa-ku, Tokyo (JP); Aoyagi, Takaaki, Fujisawa-shi, Kanagawa (JP); Umezawa, Yoji, Meguro-ku, Tokyo (JP); Morishima, Hajime, c/o Banyu Pharm. Co. Ltd., Tsukuba-shi, Ibaraki (JP); Naitoh, Kyozo, c/o Banyu Pharm. Co. Ltd., Tsukuba-shi, Ibaraki (JP); Okuyama, Akira, c/o Banyu Pharm. Co. Ltd., Tsukuba-schi, Ibaraki (JP)
(74) Representative: Reitzner, Bruno, Dr.

(57) **Abstract**

A compound of general formula [I]: 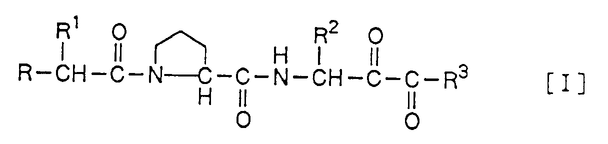
or its hydrates wherein R represents hydrogen atom, a lower alkanoyl amino group, a lower alkyloxy carbonylamino group or an aralkyloxy carbonylamino group, R¹ and R² may be identical or different from each other and each represents a lower alkyl group, and R³ represents a C₃-C₆ lower alkyl group, a cycloalkyl lower alkyl group or an aryl lower alkyl group. The compound is effective as an elastase inhibitor and a therapeutic agent.

## Description

### FIELD OF THE INVENTION

The present invention relates to an elastase inhibitor, and in particular to compounds of general formula [I] and to pharmaceutical compositions containing them.

### BACKGROUND OF THE INVENTION

Many elastase inhibitors have been known However, most of them are irreversible inhibitors which bind to hydroxy groups of serine residue in the active site irreversibly to deactivate and denaturize the enzymes. Thus there is fear of side-effects. On the other hand, as reversible inhibitors, substrate-like aldehydes, ketones, boronic acids or elastatinals derived from natural sources (Journal of Antibiotics, 28, 337-339, 1975), elasnin (Journal of The American Chemical Society, 101, 4386-4388, 1979) or the like are known.

In spite of the expectation of elastase inhibitors as therapeutic agents for diseases such as acute arteritis, pulmonary emphysema, arterial sclerosis, articular rheumatism and metastasis and invasion of carcinoma, the inhibitors are not used as drugs since the problems of intracorporeal kinetics, side effects or the like have not been dissolved.

### SUMMARY OF THE INVENTION

The object of the present invention is to provide a new elastase inhibitor having a new structure.

The another object of the present invention is to provide a new elastase inhibitor which is useful in the treatment of acute arteritis, pulmonary emphysema, arterial sclerosis, articular rheumatism and metastasis and invasion of carcinoma.

The still another object of the present invention is to provide a new elastase inhibitor which may not cause side-effects.

The present inventors have discovered that the diketone derivatives of the following general formula [I] inhibit elastase activity potently and have completed the present invention.

Thus the present invention provides compounds of general formula [I] 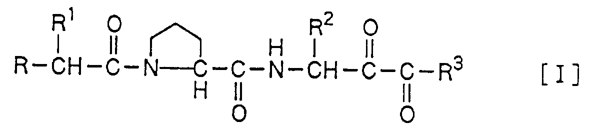
or its hydrates, wherein
R represents hydrogen atom, a lower alkanoylamino group, a lower alkyloxycarbonylamino group or an aralkyloxycarbonylamino group,
R¹ and R² may be identical or different from each other and each represents a lower alkyl group,
R³ represents a C₃-C₆ lower alkyl group, a cycloalkyl lower alkyl group or an aryl lower alkyl group.

### DETAILED DESCRIPTION OF THE INVENTION

In the specification the following abbreviations are used:
- THF: tetrahydrofuran
- DMF: N,N-dimethylformamide
- DMSO: dimethylsulfoxide
- DCC: N,N'-dicyclohexylcarbodiimide
- EDCI·HCl: 1-ethyl-3-(3-dimethylaminopropyl) carbodiimide·hydrochloride
- HOBT·H₂O: 1-hydroxy-1H-benzotriazole·monohydrate
- HOSu: N-hydroxysuccinimide
- DPPA: diphenylphosphorylazide
- PCC: pyridinium chlorochromate
- PDC: pyridinium dichromate
The definitions of the terms used in the specification and the examples of them will be explained.

The term "lower" in the specification means a group to which the term is attached having 6 or less carbon atoms unless otherwise stated.

The term lower alkyl group means a linear or branched alkyl group having one to six carbon atoms and may include methyl group, ethyl group, propyl group, isopropyl group, butyl group, isobutyl group, sec-butyl group, tert-butyl group, pentyl group, isopentyl group, neopentyl group, hexyl group, isohexyl group.

The term lower alkanoylamino group means an amino group substituted by a linear or branched alkanoyl group having one to seven carbon atoms and may include acetylamino group, propionylamino group, butyrylamino group, isobutyrylamino group, valerylamino group, isovalerylamino group, hexanoylamino group, isohexanoylamino group.

The term lower alkyloxycarbonylamino group means an amino group substituted by an alkyloxycarbonyl group having the lower alkyl group defined above and may include methyloxycarbonylamino group, ethyloxycarbonylamino group, isopropyloxycarbonylamino group, butyloxycarbonylamino group, isobutyloxycarbonylamino group, sec-butyloxycarbonylamino group, pentyloxycarbonylamino group, isopentyloxycarbonylamino group, neopentyloxycarbonylamino group, and hexyloxycarbonylamino group.

The term aralkyloxycarbonylamino group means an amino group substituted by an aralkyloxycarbonyl group having an aralkyl group having six to ten carbon atoms and may include p-methoxybenzyloxycarbonylamino group, p-nitrobenzyloxycarbonylamino group and benzyloxycarbonylamino group.

The term cycloalkyl lower alkyl group means an alkyl group substituted by a cycloalkyl group having three to six carbon atoms and may include cyclopropylmethyl group, 2-cyclopropylethyl group, cyclobutylmethyl group, 2-cyclobutylethyl group, cyclopentylmethyl group, 2-cyclopentylethyl group, cyclohexylmethyl group and 2-cyclohexylethyl group.

The term aryl lower alkyl group means a lower alkyl group substituted by an aryl group having six to ten carbon atoms and may include benzyl group, phenethyl group, 1-naphthylmethyl group, 2-naphthylmethyl group, 1-naphthylethyl group and 2-naphthylethyl group.

In order to describe the compounds of the present invention of general formula [I] more concretely, the symbols used in the general formula [I] will be explained with preferable examples.

In the formula, R represents hydrogen atom, a lower alkanoylamino group, a lower alkyloxycarbonylamino group or an aralkyloxycarbonylamino group. The lower alkanoylamino group may include acetylamino group, propionylamino group and butyrylamino group. Among them acetylamino group is preferable. The lower alkyloxy carbonylamino group may include methoxycarbonylamino group, ethoxycarbonylamino group, propionyloxycarbonylamino group, butoxycarbonylamino group, isobutoxycarbonylamino group, sec-butoxycarbonylamino group, tert-butoxycarbonylamino group, pentyloxycarbonylamino group and isopentyloxycarbonylamino group. Among them tert-butoxycarbonylamino group is preferable. The aralkyloxycarbonylamino group may include p-methoxybenzyloxy carbonylamino group, p-nitorobenzyloxycarbonylamino group and benzyloxycarbonylamino group. Among them benzyloxycarbonylamino group is preferable.

In the formula, R¹ represents a lower alkyl group and may include a linear or branched alkyl group having one to six carbon atoms such as methyl group, ethyl group, propyl group, isopropyl group, butyl group, isobutyl group, sec-butyl group, tert-butyl group, pentyl group, isopentyl group, neopentyl group, hexyl group and isohexyl group. Among them, methyl group, butyl group, isobutyl group and sec-butyl group are preferable.

In the formula, R² represents a lower alkyl group and may include a linear or branched alkyl group having one to six carbon atoms such as methyl group, ethyl group, propyl group, isopropyl group, butyl group, isobutyl group, sec-butyl group, tert-butyl group, pentyl group, isopentyl group, neopentyl group, hexyl group and isohexyl group. Among them, methyl group, propyl group, isopropyl group, butyl group and isobutyl group are preferable.

In the formula, R³ represents a C₃-C₆ lower alkyl group, cycloalkyl lower alkyl group or aryl lower alkyl group. The lower alkyl group may include a linear or branched alkyl group having three to six carbon atoms such as propyl group, isopropyl group, butyl group, isobutyl group, sec-butyl group, tert-butyl group, pentyl group, isopentyl group, neopentyl group, hexyl group and isohexyl group. Among them, butyl group, isobutyl group and sec-butyl group are preferable. The cycloalkyl lower alkyl group may include cyclopropylmethyl group, 2-cyclopropylethyl group, cyclobutylmethyl group, 2-cyclobutylethyl group, cyclopentylmethyl group, 2-cyclopentylethyl group, cyclohexylmethyl group, 2-cyclohexylethyl group. Among them, cyclohexylmethyl group and 2-cyclohexylethyl group are preferable. The aryl lower alkyl group may include benzyl group, phenethyl group, 1-naphthylmethyl group, 1-naphthylethyl group, 2-naphthylmethyl group and 2-naphthylethyl group. Among them, benzyl group and phenethyl group are preferable.

Specific compounds of general formula [I] are:
1. (4S)-4-(N-acetyl-L-leucyl-L-prolyl)amino-1-phenyl-2,3-heptanedione
2. (4S)-4-(N-isohexanoyl-L-prolyl)amino-1-phenyl-2,3-heptanedione
3. (4S)-4-(N-acetyl-L-leucyl-L-prolyl)amino-1-phenyl-2,3-pentanedione
4. (4S)-4-(N-benzyloxycarbonyl-L-leucyl-L-prolyl)amino-1-cyclohexyl-2,3-heptanedione
5. (5S)-5-(N-benzyloxycarbonyl-L-leucyl-L-prolyl)amino-1-cyclohexyl-3,4-octanedione
6. (4S)-4-(N-benzyloxycarbonyl-L-leucyl-L-prolyl)amino-1-phenyl-2,3-heptanedione
7. (4S)-4-(N-acetyl-L-alanyl-L-prolyl)amino-1-phenyl-2,3-heptanedione
8. (4S)-4-(N-acetyl-L-alanyl-L-prolyl)amino-5-methyl-1-phenyl-2,3-hexanedione
9. (4S)-4-(N-acetyl-L-alanyl-L-prolyl)amino-1-cyclohexyl-5-methyl-2,3-hexanedione
10. (5S)-5-(N-acetyl-L-alanyl-L-prolyl)amino-1-cyclohexyl-6-methyl-3,4-heptanedione
11. (4S)-4-(N-acetyl-L-leucyl-L-prolyl)amino-5-methyl-1-phenyl-2,3-hexanedione
12. (4S)-4-(N-benzyloxycarbonyl-L-alanyl-L-prolyl)amino-5-methyl-1-phenyl-2,3-hexanedione
13. (3S)-3-(N-benzyloxycarbonyl-L-alanyl-L-prolyl)amino-2-methyl-4,5-octanedione
14. (3S)-3-(N-benzyloxycarbonyl-L-alanyl-L-prolyl)amino-2-methyl-4,5-nonanedione
15. (4S)-4-(N-tert-butoxycarbonyl-L-alanyl-L-prolyl)amino-1-cyclhexyl-5-methyl-2,3-hexanedione
16. (3S)-3-(N-tert-butoxycarbonyl-L-leucyl-L-prolyl)amino-2-methyl-4,5-octanedione and
17. (6S)-6-(N-benzyloxycarbonyl-L-leucyl-L-prolyl)aminomethyl-4,5-nonanedione.

Among them, the compounds 1, 4, 5 and 6 are preferable and the compounds 4 and 6 are more preferable.

The compounds of general formula [I] of the present invention may be prepared by the following processes 1 and 2.

### [Process 1]

A proline derivative of formula [II] 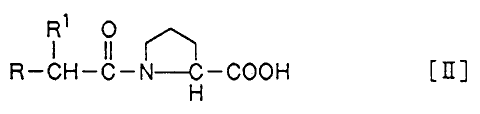
is condensed with a compound of formula [III] 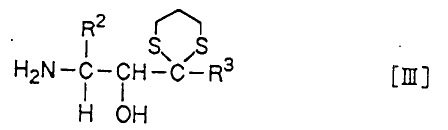
or acid addition salt thereof using a usual peptide condensing agent in a solvent, if desired in the presence of a base to prepare a compound of formula [IV] 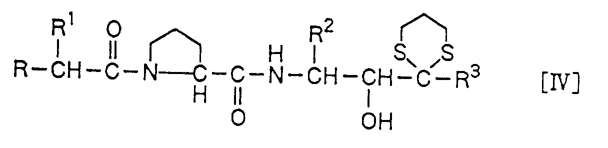
Then, the compound of formula [IV] is converted to a compound of formula [V] 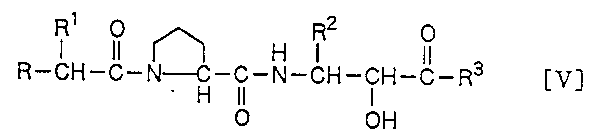
by deprotecting dithiane, and is further oxidized to prepare the compound of formula [I].

In the formulas [I], [II], [III], [IV] and [V], R, R¹, R² and R³ are as defined above.

The base used if desired in the condensation of the compound of general formula [II] and the compound of general formula [III] may include inorganic and organic bases. Organic bases such as triethylamine, piperidine, diisopropylamine and N-methylmorpholine are preferably used. The solvent used in the condensation may include DMF, THF, 1,4-dioxane, methylene chloride and chloroform. The peptide condensing agent may include one to two equivalents of DCC, EDCI·HCl and DPPA. Reagents such as HOBT·H₂O and HOSu may be used if necessary. The reaction temperature is in the range from -50° C to 50° C, preferably in the range from -15° C to 30° C and the reaction time is in the range from 30 minutes to two days.

The deprotecting agent of dithiane of general formula [IV] may include one to five equivalents of N-bromosuccinimide, ammonium cerium nitrate, ammonium thallium nitrate and mercuric chloride. The reaction solvent may include acetone, acetonitrile, aqueous acetone, aqueous acetonitrile.

The oxidation of the compound [V] to the compound [I] may be attained for example by a method using chromic acid derivatives such as one to three equivalents of PCC or PDC, a method using pyridine·sulfur trioxide complex-triethylamine and a method using DMSO such as Swern oxidation procedure and Kornblum oxidation procedure.

### [Process 2]

An aminoaldehyde derivative of formula [VI] 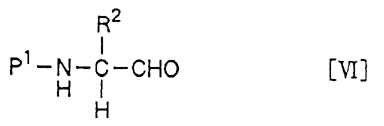
is converted to cyanohydrin derivative of formula [VII] 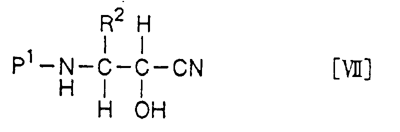
and then the hydroxy group is protected to prepare a compound of formula [VIII] 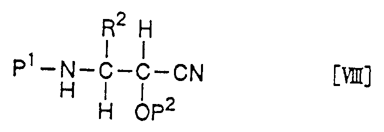
Then, the compound of formula [VIII] is reacted with a Grignard reagent of general formula [IX]

R³MgX¹ [IX]

wherein X¹ is a halogen atom to prepare a compound of general formula [X] 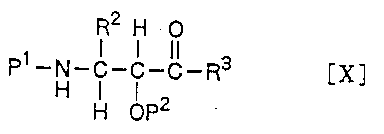
The protecting groups P¹ and P² are removed by conventional methods to afford an aminoalcohol derivative of a general formula [XI] 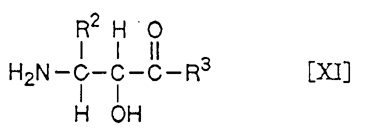
Then, the compound [XI] is condensed with a proline derivative of general formula [II] 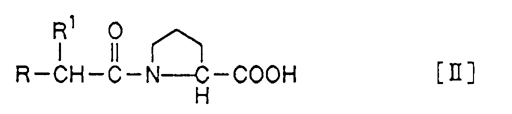
in a manner similar to Process 1 and coupling product [V] is oxidized to prepare a compound [I].

In these formulas [VI] to [XI], [I] and [II], P¹ represents a protective group of an amino group, P² represents a protective group of a hydroxy group, X¹ represents a halogen atom and R, R¹, R² and R³ are as defined above.

The method for preparing the starting materials used in the above Processes 1 and 2 will be explained.

The proline derivative [II] which is a starting material used in the above process 1 and 2 may be prepared by acylating proline by a corresponding acylating agent when R represents H. When R represents a lower alkanoylamino group, aralkyloxycarbonylamino group, the proline derivative [II] may be prepared by condensing the corresponding N-substituted amino acid and proline or C-protected proline by a usual condition of peptide condensation, i.e. the active ester method, the azide method, the mixed carbonic anhydride method, the carbodiimide method, the imidazole method, the DPPA method, Woodward's method or condensation method by oxidation/reduction system, and then if required deprotecting the compound.

The dithiane derivative [III] may be prepared by a conventional method. First a compound of formula [XII] 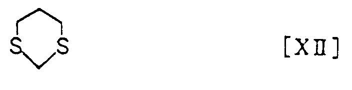
is treated with n-butyllithium and is reacted with the compound of formula [XIII]

R³X² [XIII]

wherein X² is a leaving group, to obtain a compound of general formula [XIV] 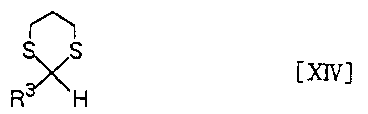
and the compound [XIV] is condensed with an N-protected aminoaldehyde of general formula [VI] 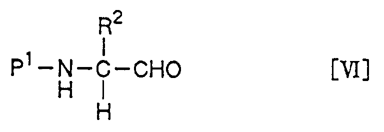
in the presence of n-butyllithium to afford a compound of general formula [XV] 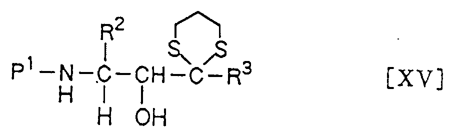
and removing the protective group by a general deprotection method of amino group.

Among the starting materials used in the Process 2, the compound [VII] may be prepared by a known process to prepare cyanohydrins, for example by reacting a compound [VI] and one to five equivalents of sodium sulfite and one to five equivalents of sodium cyanide in a mixed solvent of water and ethyl acetate at temperature in the range from 0 to 50° C, preferably 15 to 30° C for 30 minutes to two days.

In these formulas R², R³, P¹ and P² are as defined above.

As a protective group P¹ of an amino group, a conventional group such as a lower alkoxycarbonyl group such as tert-butoxycarbonyl group, and an arylmethoxycarbonyl group such as benzyloxycarbonyl group may be used. As a protective group of hydroxy group, a conventional group such as a lower alkyl silyl group such as trimethylsilyl group, tert-butyldimethylsilyl group, and arylmethyl group such as benzyl group may be used. As a protective group of carboxylic acid, a conventional group such as a lower alkyl group such as methyl group, ethyl group, and an aryl lower alkyl group such as benzyl group may be used.

In addition, as a leaving group, a conventional group such as a halogen atom, methansulfonyloxy group or p-toluenesulfonyloxy group may be used.

### Elastase inhibition activity

The inhibition activities to human leukocyte elastase were determined as follows:
Each 1µl of DMSO solution of the compound and DMSO (for reference) were added to each 250µl of 0.2M tris buffer solutions (pH8.5) containing 40µM N-methoxy succinyl-L-alanyl-L-alanyl-L-prolyl-L-valine 4-methylcoumaryl-7-amide and 1M sodium chloride, and the mixtures were pre-incubated at 37° C for three minutes. Then 50µl of 0.2M tris buffer solutions (pH8.5) containing 0.2µg/ml human leukocyte elastase were added to the mixtures and the mixtures were incubated at 37° C for 20 minutes. Each 1ml of 2M acetic acid aqueous solutions were added to the mixture and the reactions were stopped and then fluorescences of the supernatants were measured and the activities were calculated. As the results, IC₅₀ values were as follows: the compound 6 = 0.78µM, the compound 4 = 6.32µM, the compound 1 = 8.48µM and the compound 5 = 9.94µM.

Thus the compounds of the present invention show excellent elastase inhibition activities and are very useful in the medical field. In particular, the compounds of the invention may be therapeutic agents for acute arteritis, pulmonary emphysema, arterial sclerosis or inflammation. The compounds of the present invention may be used alone or in combination with another therapeutic agents when the compounds are used as therapeutic agents for these disorders.

The compounds of the present invention may be mixed with one or a number of known solid or liquid diluents or carriers and may be used in the pharmaceutical compositions which are suitable for parenteral administration, oral administration or external administration. The pharmaceutical compositions may include solutions such as injections, inhalations, syrups or emulsions, solids such as tablets, capsules or granules and preparations for external use such as ointments and suppositories. These preparations may include, if desired, one or a plurality of conventional additives such as adjuvants, preservatives, humectants, emulsifying agents, absorption promoters or surface surfactants. The additives may include sterile water for injection, Ringer's injection, glucose, sucrose syrups, gelatin, edible oils, cacao butter, ethylene glycol, sucrose, corn starch, magnesium stearate or talc.

The doses of the compounds of the present invention as an elastase inhibitor may be determined depending on the way of administration, the age, the weight and the condition of the patient to be treated. The dose of the compound is generally about 0.1 to 100mg/kg body weight in the case of oral administration for an adult, and the dose is about 0.01 to 10mg/kg body weight in the case of parenteral administration for an adult.

The compounds of the present invention are expected to be used as therapeutic agents for acute arteritis, pulmonary emphysema, arterial sclerosis, articular rheumatism and metastasis and invasion of carcinoma since the compounds inhibit elastase in human leukocyte strongly.

The present invention is illustrated by the following non-limitative Examples.

### EMBODIMENTS

### Example 1

### Preparation of (4S)-4-(N-acetyl-L-leucyl-L-prolyl)amino-1-phenyl-2,3-heptanedione (Compound 1)

### Step 1: preparation of (4S)-4-tert-butoxycarbonylamino-3-hydroxy-1-phenyl-2-heptanone trimethylene dithioacetal

0.7ml of n-butyllithium (1.6M n-hexane solution) was added to a solution of 612mg of 2-benzyl-1,3-dithiane in 3ml of absolute THF at -78° C and the mixture was stirred at -15° C for 40 minutes. A solution of 556.7mg of N-tert-butoxycarbonylnorvalinal (prepared by a known method as described in Chem. Pharm. Bull., 30, 1921-1924,1982) in 3ml of THF was added to the mixture and stirred for 15 hours. 20ml of saturated ammonium chloride aqueous solution was added to the reaction solution and the mixture was extracted with 20ml of ethyl acetate three times. The organic layer was washed with brine, dried, and concentrated under reduced pressure. The resulting residue was chromatographed (Wako-gel™ C-200 20g, chloroform) and the fraction containing the product was concentrated, and was further purified by a silica gel column chromatography (Wako-gel™ C-200 10g, hexane/ethyl acetate=8:1) to give 102.4mg of the title compound.
FABMS [M+H]⁺=412

### Step 2: Preparation of (4S)-4-(N-acetyl-L-leucyl-L-prolyl)amino-3-hydroxy-1-phenyl-2-heptanone trimethylene dithioacetal

0.5ml of trifluoroacetic acid was added to a solution of 191.9mg of the compound of step 1 in 2ml of methylene chloride and the mixture was stirred at room temperature for 30 minutes. After concentrating the reaction mixture to dryness, 115 mg of N-acetyl-L-leucyl-L-proline was added and the mixture was dissolved in 4ml of absolute DMF. 91µl of DPPA and 118µl of triethylamine were added at -15° C and stirred overnight and 40ml of ethyl acetate was added to the reaction mixture. The reaction mixture was washed with 1N hydrochloric acid, water, saturated sodium bicarbonate aqueous solution and brine and the organic layer was dried and concentrated. The resulting residue was chromatographed (Wako-gel™ C-200 15g, chloroform/methanol=100:1 then 50:1) to give 188.3mg of the title compound.
FABMS [M+H]⁺=564

### Step 3: Preparation of 2-[(2S)-2-(N-acetyl-L-leucyl-L-prolyl)aminovaleryl]-2-benzyl-1,3-dithiane

0.8ml of absolute acetic acid was added to a solution of 108.5mg of the compound of step 2 in 1.2ml of absolute DMSO and the mixture was stirred at room temperature overnight. After extraction with 40ml of ethyl acetate, the organic layer was washed with saturated sodium bicarbonate aqueous solution and brine, was dried and concentrated. The resulting residue was chromatographed (Wako-gel™ C-200 10g, chloroform/methanol=200:1 then 100:1) to give 72.6mg of the title compound.
FABMS [M+H]⁺=562

### Step 4: Preparation of (4S)-4-(N-acetyl-L-leucyl-L-prolyl)amino-1-phenyl-2,3-pentanedione

A solution of 159mg of N-bromosuccinimide in 1.3ml of 97% aqueous acetone was added to a solution of 62.8mg of the compound of step 3 in 0.5ml of 97% aqueous acetone at 0° C and the mixture was stirred for 30 seconds. After adding saturated sodium sulfite aqueous solution to the mixture, the product was extracted into ethyl acetate, the combined organic layer was washed with saturated sodium bicarbonate aqueous solution, water and brine. After drying and concentrating the organic layer, the residue was chromatographed (Wako-gel™ C-200 5g, chloroform/methanol=200:1 then 100:1) to give 12.2mg of the title compound.
FABS [M+H]⁺=472

### Example 2

### Preparation of (4S)-4-(N-benzyloxycarbonyl-L-leucyl-L-prolyl)amino-1-cyclohexyl-2,3-heptanedione (Compound 4)

### Step 1: Synthesis of (4S)-4-tert-butoxycarbonylamino-1-cyclohexyl-3-hydroxy-2-heptanone trimethylenedithioacetal

1.2ml of n-butyllithium (1.59M pentane solution) was added to a solution of 393.7mg of 2-cyclohexylmethyl-1,3-dithiane in 2ml of absolute THF at -78° C and the mixture was stirred at -15° C for one hour. A solution of 201.3mg of N-tert-butoxycarbonylnorvalinal in 2ml of THF was added to the mixture and stirred for 24 hours. 20ml of saturated ammonium chloride aqueous solution was added to the reaction mixture and the product was extracted with 20ml of ethyl acetate three times. The organic layer was washed with brine, then dried and concentrated under reduced pressure. The residue was chromatographed (Wako-gel™ C-200 20g, chloroform). After concentrating a fraction containing the object material, the fraction was further purified by a silica gel column chromatography (Wako-gel™ C-200 10g, hexane/ethyl acetate=8:1) to give 82.6mg of the title compound.
FABMS [M+H]⁺=418

### Stage 2: Preparation of (4S)-4-(N-benzyloxycarbonyl-L-leucyl-L-prolyl)amino-1-cyclohexyl-3-hydroxy-2-heptanone-2,2-bis-(1,3-dithiopropyl)ketal

1.0ml of trifluoroacetic acid was added to a solution of 494mg of the compound of step 1 in 3ml of methylene chloride and the mixture was stirred at room temperature for one hour. After concentrating the reaction solution to dryness, the residue was dissolved in 7ml of absolute DMF and 165µl of triethylamine was added. The solution was added to a solution of 391.7mg of N-benzyloxycarbonyl-L-leucyl-L-proline, 250µl of DPPA and 118µl of triethylamine in 3ml of absolute DMF at -15° C and the mixture was stirred overnight. 40ml of ethyl acetate was added to the reaction mixture and the organic layer was separated and washed with 1N hydrochloric acid, water, saturated sodium bicarbonate aqueous solution and brine. The organic layer was then dried and concentrated. The residue was purified by a silica gel column chromatography (Wako-gel™ C-200 15g, chloroform/methanol=100:1 then 50:1) to give 289mg of the title compound.
FABMS [M+H]⁺=662

### Step 3: Preparation of (4S)-4-(N-benzyloxycarbonyl-L-leucyl-L-prolyl)amino-1-cyclohexyl-3-hydroxy-2-heptanone

A solution of 47mg of N-bromosuccinimide in 1.0ml of 97% aqueous acetone was added to a solution of 21.9mg of the compound of step 3 in 0.5ml of 97% aqueous acetone at 0° C and the mixture was stirred for 30 minutes. 10% sodium sulfite aqueous solution was added to the mixture and the product was extracted with ethyl acetate. The organic layer was then washed with saturated sodium bicarbonate aqueous solution, water and brine successively. The organic layer was dried and concentrated and the residue was chromatographed (Wako-gel™ C-200 5g, chloroform/methanol=200:1 then 100:1) to give 12.2mg of the title compound.
FABMS [M+H]⁺=572

### Step 4: Preparation of (4S)-4-(N-benzyloxycarbonyl-L-leucyl-L-prolyl)amino-1-cyclohexyl-2,3-heptanedione

25µl Of DMSO was added to a solution of 15µl of oxalyl chloride in 0.3ml of absolute methylene chloride at -78° C and the mixture was stirred for ten minutes. A solution of 45.2mg of the compound of step 3 in 1.0ml of absolute methylene chloride was added to the reaction mixture and was stirred at -78° C for 40 minutes and then -20° C for 50 minutes. After cooling the mixture to -78° C again, 37µl of triethylamine was added to the mixture and was stirred for 25 minutes at -78° C and then for 30 minutes at -20° C. 30ml of ethyl acetate was added to the reaction mixture and the organic layer was separated and washed with 1N hydrochloric acid, water, saturated sodium bicarbonate aqueous solution and brine, the mixture was dried and concentrated. The resulting residue was chromatographed (Wako-gel™ C-200 3g, chloroform/methanol=100:1 then 50:1) to give 23.2mg of the title compound.
FABMS [M+H]⁺=570

### Example 3

### Synthesis of (3S)-3-(N-benzyloxycarbonyl-L-alanyl-L-prolyl)amino-2-methyl-4,5-octanedione (Compound 13)

### Step 1: Preparation of (3S)-3-tert-butoxycarbonylamino-2-hydroxyhexanenitrile

1.29g of N-tert-butoxycarbonyl-L-valinal was dissolved in 6ml of ethyl acetate and a solution of 668mg of sodium sulfite in 5ml of water was added and the mixture was stirred at room temperature overnight. After diluting the mixture with 20ml of ethyl acetate, a solution of 315mg of sodium cyanide dissolved in 8ml of water was added dropwise to the mixture for two hours. Then the mixture was stirred at room temperature overnight. 40ml of ethyl acetate was added to the reaction mixture. The organic layer was washed with water and then saturated sodium bicarbonate aqueous solution, and the organic layer was then dried and concentrated. The resulting residue was chromatographed (Wako-gel™ C-200 20g, hexane/ethyl acetate=5:1) to give 643mg of the title compound.
FABMS [M+H]⁺=229

### Step 2: Preparation of (3S)-3-tert-butoxycarbonylamino-2-tert-butyldimethylsiloxyhexanenitrile

1.02ml of triethylamine, 1.26g of tert-butyldimethyl chlorosilane and 383mg of dimethylaminopyridine were added to a solution of 1.15g of the compound obtained in the above step 1 in 16ml of methylene chloride and the mixture was stirred at room temperature for two days. The reaction mixture was extracted with 300ml of ethyl acetate, and the organic layer was washed with 1N hydrochloric acid, water, saturated sodium bicarbonate aqueous solution, water and brine, and the organic layer was dried and concentrated. The residue was chromatographed (Wako-gel™ C-200 20g, hexane/ethyl acetate=5:1) and a fraction containing the product was concentrated to dryness to give 1.07g of the title compound.
FABMS [M+H]⁺=343

### Step 3: Preparation of (3S)-3-tert-butoxycarbonylamino-4-tert-butyldimethylsiloxy-2-methyl-5-octanone

8ml of propylmagnesium bromide (0.95M THF solution) was added to a solution of 431.8mg of the compound of step 2 in 5ml of absolute THF and the mixture was stirred at room temperature overnight. To the reaction mixture, was added saturated ammonium chloride aqueous solution, and the mixture was extracted with 30ml of ethyl acetate. The combined organic layer was washed with brine, dried and concentrated under reduced pressure. The residue was chromatographed (Wako-gel™ C-200 20g, hexane/ethyl acetate=10:1) to give 244mg of the title compound.
FABMS [M+H]⁺=388

### Step 4: Preparation of (3S)-3-tert-butoxycarbonylamino-4-hydroxy-2-methyl-5-octanone

1ml of tetra-n-butylammonium fluoride (1N-THF solution) was added to a solution of 175.8mg of the compound of step 3 in 1ml of absolute THF and the mixture was stirred at 0° C for one hour. The reaction mixture was extracted with 40ml of ethyl acetate and the organic layer was washed with brine, dried and concentrated. The residue was purified by a silica gel column chromatography (wako-gel™ C-200 20g, hexane/ethyl acetate=5:1) to give 99.7mg of the title compound.
FABMS [M+H]⁺=274

### Step 5: Preparation of (3S)-3-(N-benzyloxycarbonyl-L-alanyl-L-prolyl)amino-3-hydroxy-2-methyl-5-octanone

99.7mg of the compound of step 4 was dissolved in 2ml of 4N hydrogen chloride dioxane solution and the mixture was stirred at room temperature for one hour. The reaction mixture was concentrated to dryness, the residue was dissolved in 2ml of absolute DMF. 126mg of N-benzyloxycarbonyl-L-alanyl-L-proline, 100µl of triethylamine and 90µl of DPPA were added to the mixture at -15° C and the mixture was stirred overnight at room temperature. The reaction mixture was extracted with 30ml of ethyl acetate and the organic layer was washed with 1N hydrochloric acid, water then brine, dried and concentrated. The residue was chromatographed (wako-gel™ C-200 10g, chloroform/methanol=100:1, 50:1 then 20:1) to give 40mg of the title compound.
FABMS [M+H]⁺=476

### Step 6: Preparation of (3S)-3-(N-benzyloxycarbonyl-L-alanyl-L-prolyl)amino-2-methyl-4,5-octanedione (Compound 13)

13µl of DMSO was added to a solution of 8µl of oxalyl chloride in 0.2ml of absolute methylene chloride at -78° C and the mixture was stirred for 20 minutes. To the reaction mixture, a solution of 14.2mg of the compound of step 5 in 0.5ml of absolute methylene chloride was added and was stirred at -15° C for 1.5 hours. 25µl of triethylamine was added at -78° C and the mixture was stirred at -15° C for 20 minutes. The reaction mixture was extracted with 30ml of ethyl acetate and the organic layer was washed with 1N hydrochloric acid, water and brine, dried and concentrated. The residue was purified by a silica gel column chromatography (Wako-gel™ C-200 3g, chloroform/methanol=100:1 then 50:1) to give 8.6mg of the title compound.
FABMS [M+H]⁺=474

The compounds of Examples 4 to 10 were prepared in a manner similar to Example 1.

### Example 4

### (4S)-4-(N-isohexanoyl-L-prolyl)amino-1-phenyl-2,3-heptanedione (Compound 2)

FABMS [M+H]⁺=415

### Example 5

### (4S)-4-(N-acetyl-L-leucyl-L-prolyl)amino-1-phenyl-2,3-pentanedione (Compound 3)

FABMS [M+H]⁺=444

### Example 6

### (4S)-4-(N-benzyloxycarbonyl-L-leucyl-L-prolyl)amino-1-phenyl-2,3-heptanedione (Compound 6)

FABMS [M+H]⁺=564

### Example 7

### (4S)-4-(N-acetyl-L-alanyl-L-prolyl)amino-1-phenyl-2,3-heptanedione (Compound 7)

FABMS [M+H]⁺=430

### Example 8

### (4S)-4-(N-acetyl-L-alanyl-L-prolyl)amino-5-methyl-1-phenyl-2,3-hexanedione (Compound 8)

FABMS [M+H]⁺=430

### Example 9

### (4S)-4-(N-acetyl-L-leucyl-L-prolyl)amino-5-methyl-1-phenyl-2,3-hexanedione (Compound 11)

FABMS [M+H]⁺=472

### Example 10

### (4S)-4-(N-benzyloxycarbonyl-L-alanyl-L-prolyl)amino-5-methyl-1-phenyl-2,3-hexanedione (Compound 12)

FABMS [M+H]⁺=522
The compounds of Examples 11 to 14 were prepared in a manner similar to Example 2.

### Example 11

### (5S)-5-(N-benzyloxycarbonyl-L-leucyl-L-prolyl)amino-1-cyclohexyl-3,4-octanedione (Compound 5)

FABMS [M+H]⁺=584

### Example 12

### (4S)-4-(N-acetyl-L-alanyl-L-prolyl)amino-1-cyclohexyl-5-methyl-2,3-hexanedione (Compound 9)

FABMS [M+H]⁺=436

### Example 13

### (5S)-5-(N-acetyl-L-alanyl-L-prolyl)amino-1-cyclohexyl-6-methyl-3,4-heptanedione (Compound 10)

FABMS [M+H]⁺=450

### Example 14

### (4S)-4-(N-tert-butoxycarbonyl-L-alanyl-L-prolyl)amino-1-cyclohexyl-5-methyl-2,3-hexanedione (Compound 15)

FABMS [M+H]⁺=494
The compounds of Example 15 to 17 were prepared in a manner similar to Example 3.

### Example 15

### (3S)-3-(N-benzyloxycarbonyl-L-alanyl-L-prolyl)amino-2-methyl-4,5-nonanedione (Compound 14)

FABMS [M+H]⁺=488

### Example 16

### (3S)-3-(N-tert-butoxycarbonyl-L-leucyl-L-prolyl)amino-2-methyl-4,5-octanedione (Compound 16)

FABMS [M+H]⁺=482

### Example 17

### (6S)-6-(N-benzyloxycarbonyl-L-leucyl-L-prolyl)amino-2-methyl-4,5-nonanedione (Compound 17)

FABMS [M+H]⁺=530

## Claims

1. A compound of general formula [I]: 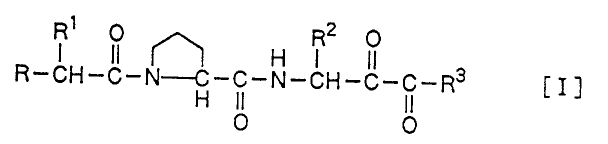 or its hydrates wherein
R represents hydrogen atom, a lower alkanoyl amino group, a lower alkyloxycarbonylamino group or an aralkyloxycarbonylamino group,
R¹ and R² may be identical or different from each other and each represents a lower alkyl group, and
R³ represents a C₃-C₆ lower alkyl group, a cycloalkyl lower alkyl group or an aryl lower alkyl group.

2. A compound according to claim 1 or its hydrates wherein R represents hydrogen atom, acetylamino group, propionylamino group, butyrylamino group, methoxycarbonylamino group, ethoxycarbonylamino group, propionyloxycarbonylamino group, butoxycarbonylamino group, isobutoxycarbonylamino group, sec-butoxycarbonylamino group, tert-butoxycarbonylamino group, pentyloxycarbonylamino group, isopentyloxycarbonylamino group, p-methoxybenzyloxycarbonylamino group, p-nitrobenzyloxycarbonylamino group or benzyloxycarbonylamino group.

3. A compound according to claim 1 or its hydrates wherein R¹ represents methyl group, ethyl group, propyl group, isopropyl group, butyl group, isobutyl group, sec-butyl group, tert-butyl group, pentyl group, isopentyl group, neopentyl group, hexyl group or isohexyl group.

4. A compound according to claim 1 or its hydrates in which R² represents methyl group, ethyl group, propyl group, isopropyl group, butyl group, isobutyl group, sec-butyl group, tert-butyl group, pentyl group, isopentyl group, neopentyl group, hexyl group or isohexyl group.

5. A compound according to claim 1 or its hydrates in which R³ represents a cycloalkyl lower alkyl group or an aryl lower alkyl group.

6. A compound according to claim 1 or its hydrates in which R³ represents a cycloalkyl lower alkyl group.

7. A compound according to claim 1 or its hydrates in which R³ represents cyclopropylmethyl group, 2-cyclopropylethyl group, cyclobutylmethyl group, 2-cyclobutylethyl group, cyclopentylmethyl group, 2-cyclopentylethyl group, cyclohexylmethyl group or 2-cyclohexylethyl group.

8. A compound according to claim 1 or its hydrates wherein R³ represents an aryl lower alkyl group.

9. A compound according to claim 1 or its hydrates in which R³ represents benzyl group, phenethyl group, 1-naphthylmethyl group, 1-naphthylethyl group, 2-naphthylmethyl group or 2-naphthylethyl group.

10. A compound according to claim 1 in which the compound is :
(4S)-4-(N-acetyl-L-leucyl-L-prolyl)amino-1-phenyl-2,3-heptanedione,
(4S)-4-(N-isohexanoyl-L-prolyl)amino-1-phenyl-2,3-heptanedione,
(4S)-4-(N-acetyl-L-leucyl-L-prolyl)amino-1-phenyl-2,3-pentanedione,
(4S)-4-(N-benzyloxycarbonyl-L-leucyl-L-prolyl)amino-1-cyclohexyl-2,3-heptanedione,
(5S)-5-(N-benzyloxycarbonyl-L-leucyl-L-prolyl)amino-1-cyclohexyl-3,4-octanedione,
(4S)-4-(N-benzyloxycarbonyl-L-leucyl-L-prolyl)amino-1-phenyl-2,3-heptanedione,
(4S)-4-(N-acetyl-L-alanyl-L-prolyl)amino-1-phenyl-2,3-heptanedione,
(4S)-4-(N-acetyl-L-alanyl-L-prolyl)amino-5-methyl-1-phenyl-2,3-hexanedione,
(4S)-4-(N-acetyl-L-alanyl-L-prolyl)amino-1-cyclohexyl-5-methyl-2,3-hexanedione,
(5S)-5-(N-acetyl-L-alanyl-L-prolyl)amino-1-cyclohexyl-6-methyl-3,4-heptanedione,
(4S)-4-(N-acetyl-L-leucyl-L-prolyl)amino-5-methyl-1-phenyl-2,3-hexanedione,
(4S)-4-(N-benzyloxycarbonyl-L-alanyl-L-prolyl)amino-5-methyl-1-phenyl-2,3-hexanedione,
(3S)-3-(N-benzyloxycarbonyl-L-alanyl-L-prolyl)amino-2-methyl-4,5-octanedione,
(3S)-3-(N-benxyloxycarbonyl-L-alanyl-L-prolyl)amino-2-methyl-4,5-nonanedione,
(4S)-4-(N-tert-butoxycarbonyl-L-alanyl-L-propyl)amino-1-cyclohexyl-5-methyl-2,3-hexanedione,
(3S)-3-(N-tert-butoxycarbonyl-L-leucyl-L-prolyl)amino-2-methyl-4,5-octanedione, or
(6S)-6-(N-benzyloxycarbonyl-L-leucyl-L-prolyl)amino-2-methyl-4,5-nonanedione.

11. A pharmaceutical composition containing a compound of general formula [I] as claimed in claim 1 together with a pharmaceutically acceptable diluent or carrier.

12. The use of a compound of general formula [I] as claimed in claim 1 as an elastase inhibitor.

13. The use of a compound of general formula [I] as claimed in claim 1 as a medicament or for the manufacture of a medicament.
